# EUROPEAN PATENT SPECIFICATION

(11) **EP 3 730 110 B1**
(45) Date of publication and mention of the grant of the patent: **20.09.2023**
(21) Application number: 17935751.2
(22) Date of filing: 19.12.2017
(51) Int. Cl.: A61F 13/496, A61F 13/49

(54) **PANTS-TYPE WEARABLE ARTICLE**
HOSENARTIGES KLEIDUNGSSTÜCK
ARTICLE POUVANT ÊTRE PORTÉ DE TYPE CULOTTE

(43) Date of publication of application: 28.10.2020
(73) Proprietor: Kao Corporation, Chuo-ku, Tokyo 103-8210 (JP)
(72) Inventor: MATSUDA, Kohei, Haga-gun, Tochigi 321-3497 (JP); KAWAGUCHI, Hiroko, Haga-gun, Tochigi 321-3497 (JP); OKUDA, Yasuyuki, Haga-gun, Tochigi 321-3497 (JP)
(74) Representative: Vossius & Partner Patentanwälte Rechtsanwälte mbB
(86) International application number: PCT/JP2017/045593
(87) International publication number: WO 2019/123549

(56) References cited:
- EP-A1- 3 649 997
- WO-A1-2017/017831
- JP-A- 2009 148 465
- JP-A- 2017 012 232
- JP-A- 2017 104 197
- JP-A- 2017 131 451
- JP-A- 2017 131 451

## Description

### Technical Field

The present invention relates to a pull-on disposable diaper.

### Background Art

A pull-on garment includes a pair of side seals formed by joining both sides of a front portion disposed on the front side of a wearer during wear and both sides of a rear portion disposed on the rear side of the wearer during wear. In general, such a pull-on garment can be easily removed by tearing the side seals after the use of the pull-on garment. As the pull-on garment having such a configuration, a pull-on garment has been proposed on which joining mode and the like of the side seal is studied from the viewpoint of ease in tearing the side seal at the time of removing the pull-on garment.

For example, the applicant of the present invention has previously proposed a pull-on absorbent article where a side seal includes portions which differ in the number of constituent members, and the portion having the larger number of constituent members has a smaller fusion bonded area in the side seal (see JP 2010-115424 A). According to this pull-on absorbent article, the forming pattern of the joined regions (sealed portions) formed in the side seal is varied so that even when portions which differ in the number of constituent members are present in the side seal, a substantially uniform fusion bonding strength can be acquired. Accordingly, the side seal can be peeled with a uniform force at the time of peeling the side seal after use.

The applicant of the present invention has also previously proposed a pull-on disposable diaper where a region forming a side seal has a laminated structure formed of a plurality of sheets, and includes a plurality of portions which differ in the number of sheets laminated, and an amount of seal heat reducing agent which corresponds to the number of sheets laminated is applied between the sheets (Japanese Patent No. 5086055). According to this pull-on disposable diaper, tear of the side seal, partial peeling and the like do not occur during wear, and the side seal can be easily peeled at the time of removing the diaper.

EP 3 649 997 A1 (prior art under Art. 54(3) EPC) discloses a disposable diaper comprising a front portion and a rear portion, both sides of the front portion and both sides of the rear portion being joined with each other to form a pair of side seals, a waist opening, and a pair of leg openings. The side seals include a joined region where the front portion and the rear portion are joined with each other, and a fusion bonded region where at least either one of a plurality of constituent sheets for the front portion and a plurality of constituent sheets for the rear portion are fusion bonded. Other technology is shown in JP 2017 131451 A or JP 2017 104197 A.

### Summary of Invention

The present invention is directed to a pull-on disposable diaper as defined in independent claim 1.

Preferably, the side seals include a plurality of the joined regions arranged intermittently in a longitudinal direction of the side seal, and one or more fusion bonded regions located between the joined regions. More preferably, the total area of the fusion bonded regions in the side seal is set to 10% or more and preferably set to 150% or more, is set to 700% or less and preferably set to 450% or less, and is set to 10% or more and 950% or less and preferably set to 150% or more and 700% or less of the total area of the joined regions. The area of one fusion bonded region in the side seal may be set to 10% or more and preferably set to 50% or more, may be set to 300% or less and preferably set to 120% or less, and is set to 10% or more and 300% or less and preferably set to 50% or more and 120% or less of the area of one joined region. The number of fusion bonded regions between the joined regions disposed adjacent to each other in the longitudinal direction of the side seal may be set to 2 or more and preferably set to 3 or more, ma be set to 10 or less and preferably set to 5 or less, and may be set to 2 or more and 10 or less and preferably set to 3 or more and 5 or less.

Preferably, the side seals include the fusion bonded region on the front portion and on the rear portion, and the fusion bonded region of the front portion and the fusion bonded region of the rear portion are disposed at different positions in the width direction of the side seal. More preferably, the fusion bonded region of the front portion and the fusion bonded region of the rear portion partially overlap with each other, and the area of a portion where the fusion bonded region of the front portion and the fusion bonded region of the rear portion overlap with each other in the side seal is set to 50% or less and preferably set to 20% or less of the total area of the fusion bonded regions of the front portion and the fusion bonded regions of the rear portion in the side seal. The fusion bonded region of the front portion and the fusion bonded region of the rear portion may be aligned in the width direction of the side seal, the fusion bonded region of the front portion and the fusion bonded region of the rear portion being disposed at different positions in the width direction of the side seal.

Preferably, the side seals include a plurality of the joined regions aligned intermittently in the longitudinal direction of the side seal, and a plurality of the fusion bonded regions in a common space formed between the joined regions disposed adjacent to each other in the longitudinal direction. The fusion bonded region of the front portion and the fusion bonded region of the rear portion are preferably formed in a common space formed between the joined regions disposed adjacent to each other in the longitudinal direction of the side seal. A plurality of the fusion bonded regions may be aligned in a width direction of the side seal. The plurality of the fusion bonded regions may be aligned at equal intervals in the width direction of the side seal. An arrangement pitch of the fusion bonded regions in the width direction of the side seal may be two times or more of a length of the fusion bonded region in the width direction.

Preferably, the pull-on garment includes a portion where the joined region and the fusion bonded region overlap with each other.

Preferably, a length of the joined region in the longitudinal direction of the side seal is shorter than the length of the side seal in the width direction.

Preferably, the length L1 of the joined region in the longitudinal direction of the side seal is set to 5% or more and preferably set to 15% or more, is set to 100% or less and preferably set to 50% or less, and is set to 5% or more and 100% or less and preferably set to 15% or more and 50% or less of the length W of the side seal in the width direction. Preferably, the length L2 of the fusion bonded region in the longitudinal direction of the side seal is set to 10% or more and preferably set to 50% or more, is set to 800% or less and preferably set to 400% or less, and is set to 10% or more and 800% or less and preferably set to 50% or more and 400% or less of the length W2 of the side seal in the width direction.

The arrangement pitch of the joined regions in the longitudinal direction of the side seal is longer than a length of the joined region in the width direction of the side seal. Preferably, the arrangement pitch P1 of the joined regions in the longitudinal direction of the side seal is set to 50% or more and preferably set to 60% or more, is set to 200% or less and preferably set to 160% or less, and is set to 50% or more and 200% or less and preferably set to 60% or more and 160% or less of the length W of the side seal in the width direction of the side seal. Preferably the arrangement pitch P1 of the joined regions in the longitudinal direction of the side seal is set to 200% or less and preferably set to 160% or less of the length W1 of the joined region in the width direction of the side seal.

Preferably, the arrangement pitch P1 of the joined regions in the longitudinal direction of the side seal is set to 100% or more and preferably set to 300% or more, is set to 1000% or less and preferably set to 700% or less, and is set to 100% or more and 1000% or less and preferably set to 300% or more and 700% or less of the length L1 of the joined region in the longitudinal direction of the side seal.

Preferably, the arrangement pitch P2 of the fusion bonded regions in the longitudinal direction of the side seal is set to 10% or more and preferably set to 100% or more, is set to 600% or less and preferably set to 500% or less, and is set to 10% or more and 600% or less and preferably set to 100% or more and 500% or less of the length L2 of the fusion bonded region in the longitudinal direction of the side seal.

Preferably, the arrangement pitch P2 of the fusion bonded regions in the longitudinal direction of the side seal is set to 300% or more and preferably set to 400% or more, is set to 2000% or less and preferably set to 1400% or less, and is set to 300% or more and 2000% or less and preferably set to 400% or more and 1400% or less of the length W2 of the fusion bonded region in the width direction of the side seal.

Preferably, the arrangement pitch P2 of the fusion bonded regions in the longitudinal direction of the side seal is set to 10% or more and preferably set to 50% or more, is set to 100% or less and preferably set to 90% or less, and is set to 10% or more and 100% or less and preferably set to 50% or more and 90% or less of the arrangement pitch P1 of the joined regions in the longitudinal direction of the side seal.

Preferably, the side seals include the fusion bonded region in at least a region on a waist opening side out of two regions defined by dividing an entire side seal in the longitudinal direction into two regions.

Preferably, the side seals include the fusion bonded region at least in a region on the waist opening side out of three regions defined by dividing the entire side seal in the longitudinal direction into three regions.

Preferably, the side seals include the fusion bonded region in a region having a range of 50 mm in the longitudinal direction of the side seal from an end portion of the side seal on the waist opening side.

Preferably, an arrangement pitch of the fusion bonded regions in the longitudinal direction of the side seal is shorter than the arrangement pitch of the joined regions in the longitudinal direction of the side seal.

Preferably, the pull-on garment includes, between the constituent sheets for the front portion and between the constituent sheets for the rear portion, an elastic member which extends in a width direction of the pull-on garment, and the elastic member is disposed between the fusion bonded regions disposed adjacent to each other in a longitudinal direction of the pull-on garment. In a flat-out, uncontracted state of the pull-on garment, an arrangement pitch of the fusion bonded regions in the width direction of the pull-on garment may be shorter in a periphery of the side seal than in a portion other than the periphery of the side seal. In the flat-out, uncontracted state of the elastic member of the pull-on garment, the arrangement pitch of the fusion bonded regions in the width direction of the pull-on garment may be shorter in a region which falls within a range of 50 mm from an outer end portion of the pull-on garment in the width direction toward an inside in the width direction of the pull-on garment than in a portion other than the range.

Preferably, each of the rear portion and the front portion includes the waist elasticized portion and the below-waist lower elasticized portion and, in a portion of each of the rear portion and the front portion other than the side seals, the plurality of fusion bonded region rows are formed at intervals in the article lateral direction, each fusion bonded region row being formed of the plurality of fusion bonded regions intermittently formed to form one row along the article longitudinal direction, and in the waist elasticized portion, all fusion bonded region rows include the fusion bonded regions disposed at substantially the same positions in the article longitudinal direction.

Preferably, both the front portion and the rear portion include a pair of outer fixing regions, where the constituent sheets are joined with each other via an adhesive agent, on both sides of the article longitudinal direction center line CL, which bisects the pull-on garment in the width direction.

Preferably, the pull-on garment includes the outer cover, and the absorbent assembly fixed to the outer cover, and the pull-on garment includes the assembly side fixing region, where the constituent sheets are joined with each other via the adhesive agent, at portions in the vicinity of positions of the side edges of the absorbent assembly. The outer fixing region may entirely or partially overlap with the side seal. The waist elasticized portion may include the plurality of elastic members arranged to extend between the pair of outer fixing regions, the plurality of elastic members are fixed between the constituent sheets in each of the pair of outer fixing regions, but are fixed to none of the constituent sheets between the outer fixing regions. The below-waist lower elasticized portion may include the plurality of elastic members arranged to extend between the outer fixing region and the assembly side fixing region, the plurality of elastic members are fixed between the constituent sheets in each of the outer fixing region and the assembly side fixing region, but are fixed to none of the constituent sheets between the outer fixing region and the assembly side fixing region. Each of the front portion and the rear portion may include, as the constituent sheets, the inner sheet disposed on the skin-facing surface side and the outer sheet disposed on the non-skin-facing surface side, the outer sheet is folded back to the skin-facing surface side of the inner sheet along the peripheral edge portion of the waist opening to form a folded portion, and the folded portion covers the skin-facing surface side of the inner sheet of the waist elasticized portion, and the folded portion is joined to the skin-facing surface of one of or both the inner sheet and the absorbent assembly by an adhesive agent which is entirely or partially applied to the folded portion, but the folded portion is not fusion bonded to the fusion bonded region.

### Brief Description of Drawings

Figure 1 is a perspective view showing a use state (wear state) of a pull-on disposable diaper which is one embodiment of the present invention.
Figure 2 is a plan view of the pull-on disposable diaper shown in Figure 1 in its flat-out, uncontracted state, and is also a view as viewed from the skin-facing surface side.
Figure 3 is an enlarged cross-sectional view showing a cross section of a waist elasticized portion of the pull-on disposable diaper shown in Figure 1 taken along an article lateral direction.
Figure 4 is an enlarged cross-sectional view showing a cross section of a below-waist lower elasticized portion of the pull-on disposable diaper shown in Figure 1 taken along the article lateral direction.
Figure 5 is a schematic view showing the arrangement relationship between joined regions and fusion bonded regions in a side seal of the pull-on disposable diaper shown in Figure 1, which is not according to the present invention.
Figure 6 is a schematic view showing a state at the time of tearing the side seal of the pull-on disposable diaper shown in Figure 1.
Figures 7(a) to (g) are views showing arrangement patterns between the joined regions and the fusion bonded regions in the side seal with Fig. 7(b), (d), and (e) being according to the present invention.
Figure 8 is a schematic cross-sectional view of an apparatus which forms the side seals of the pull-on disposable diaper shown in Figure 1.

### Description of Embodiments

A pull-on garment is required to have a joining strength of side seal to an extent that breakage of the side seals does not occur during wear. Whereas, the pull-on garment is also required to have ease in tearing the side seals at the time of removing the pull-on garment. Pull-on garments disclosed in JP 2010-115424 A and Japanese Patent No. 5086055 have a room for further improvement of a joining strength of side seal.

The present invention relates to providing a pull-on garment which realizes both a joining strength of the side seal during wear and ease in tearing the side seals at the time of removing the pull-on garment.

Hereinafter, an absorbent article of the present invention will be described with reference to drawings based on a preferred embodiment.

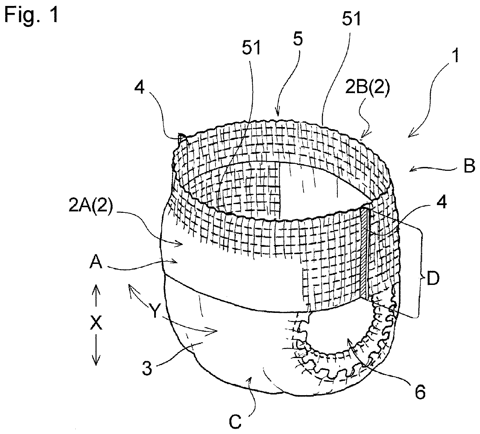

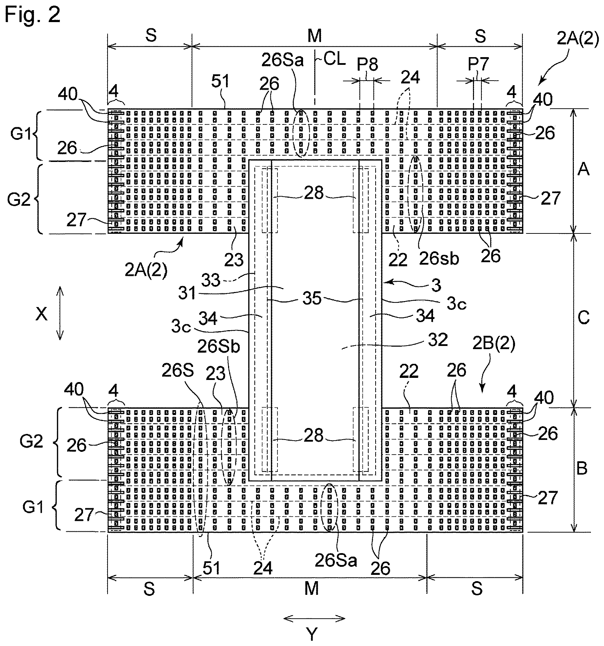

A disposable diaper 1 which is one embodiment of the present invention will be described with reference to Figure 1 and Figure 2. Hereinafter, the disposable diaper 1 is also simply referred to as the diaper 1. As shown in Figure 1, the diaper 1 of this embodiment is a pull-on disposable diaper, and includes an outer cover 2, and an absorbent assembly 3 fixed to the outer cover 2. The diaper 1 also includes a front portion A disposed on the front side of a wearer during wear, and a rear portion B disposed on the rear side of the wearer. The diaper 1 is configured such that both sides of the front portion A and both sides of the rear portion B are joined with each other to form a pair of side seals 4, 4, a waist opening 5, and a pair of leg openings 6, 6. Both sides of the front portion A are both ends of the front portion A in an article lateral direction Y, and both sides of the rear portion B are both ends of the rear portion B in the article lateral direction Y. The diaper 1 includes a crotch portion C between the front portion A and the rear portion B, the crotch portion C being disposed at the crotch of the wearer.

The outer cover 2 of the diaper 1 of this embodiment includes a front panel 2A forming the front portion A and a rear panel 2B forming the rear portion B. The front panel 2A and the rear panel 2B are joined with each other at the pair of side seals 4, 4. As shown in Figure 2, the absorbent assembly 3 extends between and is fixed to the center portion of the front panel 2A in the article lateral direction Y and the center portion of the rear panel 2B in the article lateral direction Y. Portions of the absorbent assembly 3 which respectively overlap with the front panel 2A and the rear panel 2B are entirely or partially joined with both sheet panels 2A, 2B by a known joining means, such as an adhesive agent.

The outer cover 2 may include, at both ends of the front panel or the rear panel in the article lateral direction Y, a region having a small width where the front panel 2A and the rear panel 2B are not joined, for example, a region having a width of 10 mm or less. Such a case is also included in cases where the front portion A and the rear portion B are joined with each other at both ends of the front portion A and the rear portion B in the article lateral direction Y.

As shown in Figure 2, the diaper 1 of this embodiment is formed in left-right symmetry with respect to an article longitudinal direction center line CL which extends in an article longitudinal direction X, and which bisects the diaper 1 in the article lateral direction Y. Accordingly, in the description made hereinafter, portions which are formed with left-right symmetry will be described mainly with respect to the portions on the right side in Figure 2. However, the portions on the left side also have the similar configuration except that the portions on the left side are left-right symmetric with the portions on the right side.

The article longitudinal direction X corresponds to the front-rear direction of a wearer. More specifically, the article longitudinal direction X is a direction extending from a portion disposed on the front side of the wearer to a portion disposed on the rear side through a portion disposed at the crotch. Usually, the article longitudinal direction X is aligned with the longitudinal direction of the absorbent assembly 3. Whereas, as shown in Figure 2, the article lateral direction Y is a direction orthogonal to the article longitudinal direction X with the absorbent article, such as a diaper, being in a flat-out, uncontracted state. Further, as shown in Figure 1, the article lateral direction Y is aligned with a circumferential direction of a cylindrical below-waist portion D formed by connecting the front portion A and the rear portion B with each other. The article longitudinal direction X corresponds to an upward and downward direction of the front portion A and the rear portion B during wear. Accordingly, each of the waist opening 5 side of the front portion A and the waist opening 5 side of the rear portion B is also referred to as an upper side or an upward direction, and each of the crotch portion C side of the front portion A and the crotch portion C side of the rear portion B is also referred to as a lower side or a downward direction.

The description "a flat-out, uncontracted state" of the diaper 1 means a state where side seals 4 disposed on both sides of a pull-on garment are torn to bring the pull-on garment into a flat-out state, and the pull-on garment in the flat-out state is expanded to assume a design size by uncontracting elastic members of respective portions. The design size is equal to the size of the pull-on garment when expanded in a plane in a state where the influence of the elastic members is completely eliminated. Sizes, arrangement pitches and the like of respective portions described in this specification are obtained by measuring a diaper in its flat-out, uncontracted state unless otherwise particularly described.

In this this specification, the description "skin-facing surface" means, of a front surface and a rear surface of each member, such as a topsheet 31 described later, which forms the absorbent assembly 3, a surface disposed on the wearer's skin side during wear. The description "non-skin-facing surface" means, of the front surface and the rear surface of each member, such as the topsheet 31 described later, which forms the absorbent assembly 3, a surface which is directed toward the side on a side opposite to the wearer's skin side during wear.

The diaper 1 of this embodiment is configured such that, as shown in Figure 2, each of the front panel 2A and the rear panel 2B, which form the outer cover 2, includes an outer sheet 22 forming the outer surface of the diaper, an inner sheet 23 disposed on the inner surface side of the outer sheet 22, and a plurality of filamentous elastic members 24 disposed between both sheets 22, 23 in an uncontracted state. Each of the front panel 2A and the rear panel 2B includes a waist elasticized portion G1 and a below-waist lower elasticized portion G2. Hereinafter, the waist elasticized portion G1 and the below-waist lower elasticized portion G2 are also collectively referred to as "elasticized portion G". In the diaper 1 of this embodiment, the outer sheet 22 forming the outer surface of the diaper forms an outer sheet disposed on the side remote from the wearer's skin. The inner sheet 23 forms an inner sheet of the outer cover 2, the inner sheet being disposed on the side closer to the wearer's skin than the outer sheet. This inner sheet 23 is "the inner sheet disposed on the side closer to the wearer's skin side than the outer sheet" in the present invention.

The waist elasticized portions G1 are respectively formed outward of both longitudinal ends 3a, 3b of the absorbent assembly 3 in the article longitudinal direction X of the diaper 1. The waist elasticized portions G1 are formed at the peripheral edge portion of the waist opening 5, and are disposed at the waist part of a wearer during wear. The below-waist lower elasticized portion G2 is formed at a portion of the front panel 2A below the waist elasticized portion G1 and above the lower ends of the side seals 4, and the below-waist lower elasticized portion G2 is formed at a portion of the rear panel 2B below the waist elasticized portion G1 and above the lower ends of the side seals 4.

In the waist elasticized portions G1 and the below-waist lower elasticized portions G2 in this embodiment, the outer sheet 22 and the inner sheet 23 are joined with each other by a plurality of fusion bonded regions 26 intermittently formed in the article longitudinal direction X and in the article lateral direction Y. As described above, the outer cover 2 of the diaper 1 includes the fusion bonded regions 26 which partially join the outer sheet 22 and the inner sheet 23 with each other. That is, in this embodiment, the fusion bonded regions 26 cause a plurality of constituent sheets of the front portion A, that is, the outer sheet 22 and the inner sheet 23, to be fusion bonded. The fusion bonded regions 26 also cause a plurality of constituent sheets of the rear portion B, that is, the outer sheet 22 and the inner sheet 23, to be fusion bonded.

To be more specific, as shown in Figure 2, each of the waist elasticized portion G1 and the below-waist lower elasticized portion G2 includes the fusion bonded regions 26 where the outer sheet 22 and the inner sheet 23 are fusion bonded, the fusion bonded regions 26 being intermittently formed to form one row along the article longitudinal direction X. A plurality of fusion bonded region rows 26S each of which is formed of the plurality of fusion bonded regions 26 are formed at intervals in the article lateral direction Y. Hereinafter, the fusion bonded region row in the waist elasticized portion G1 is labeled as a fusion bonded region row 26Sa, and the fusion bonded region row in the below-waist lower elasticized portion G2 is indicated as a fusion bonded region row 26Sb.

In the waist elasticized portion G1 in this embodiment, all fusion bonded region rows 26Sa include the fusion bonded regions 26 disposed at substantially the same positions in the article longitudinal direction X. Further, the plurality of elastic members 24 extend in the article lateral direction Y such that the elastic member 24 passes through spaces formed between fusion bonded regions 26 of each fusion bonded region row 26Sa, the fusion bonded regions 26 being adjacent to each other in the article longitudinal direction X. That is, in the waist elasticized portion G1, the elastic members 24 are disposed such that each elastic member 24 passes through spaces each formed between the fusion bonded regions 26, which form each fusion bonded region row 26Sa, each elastic member 24 being not fixed by the fusion bonded region 26. Also in the below-waist lower elasticized portion G2 of this embodiment, in the same manner as the waist elasticized portion G1, the elastic members 24 are disposed such that each elastic member 24 passes through spaces each formed between two fusion bonded regions 26, which form the fusion bonded region row 26Sb, each elastic member 24 being not fixed by the fusion bonded region 26.

In the diaper 1 of this embodiment, as shown in Figure 2, both of the front panel 2A and the rear panel 2B includes, on both sides of the article longitudinal direction center line CL, a pair of outer fixing regions 27 where the outer sheet 22 and the inner sheet 23 are joined with each other via an adhesive agent. Both of the front panel 2A and the rear panel 2B also include, at portions thereof in the vicinity of positions of side edges 3c of the absorbent assembly 3, assembly side fixing regions 28 where the outer sheet 22 and the inner sheet 23 are joined with each other via the adhesive agent. In each of the front panel 2A (the front portion A) and the rear panel 2B (the rear portion B), the pair of outer fixing regions 27 are formed at portions outward of the assembly side fixing regions 28 in the article lateral direction Y, that is, portions separated from the assembly side fixing regions 28 toward the side seal 4 side. More specifically, the pair of outer fixing regions 27 are formed at end portions of each of the front panel 2A and the rear panel 2B in the article lateral direction Y or at portions in the vicinity of the end portions. It is preferable that the outer fixing regions 27 entirely or partially overlap with the side seals 4.

The waist elasticized portion G1 includes the plurality of elastic members 24 arranged to extend between the pair of outer fixing regions 27. These elastic members 24 are fixed between the sheets 22, 23 in each of the pair of outer fixing regions 27. However, these elastic members 24 are fixed to neither of the sheet 22 nor the sheet 23 in a range between the outer fixing regions 27.

Whereas, the below-waist lower elasticized portion G2 includes the plurality of elastic members 24 arranged to extend between the outer fixing regions 27 and the assembly side fixing regions 28. These elastic members 24 are fixed between the sheets 22, 23 in each of the outer fixing regions 27 and the assembly side fixing regions 28. However, these elastic members 24 are fixed to neither of the sheet 22 nor the sheet 23 in ranges between the outer fixing regions 27 and the assembly side fixing regions 28.

The assembly side fixing regions 28 may be formed such that the entire assembly side fixing regions 28 overlap with the absorbent assembly 3 as shown in Figure 2. The assembly side fixing regions 28 may be formed such that each assembly side fixing region 28 straddles the side edge 3c of the absorbent assembly 3. Further, the assembly side fixing regions 28 may be formed outward of the side edges 3c of the absorbent assembly 3 in the diaper lateral direction. The mode where the entire assembly side fixing regions 28 overlap with the absorbent assembly 3 includes both the mode where the position of an outer end portion of each assembly side fixing region 28 in the diaper lateral direction and the position of the side edge 3c of the absorbent assembly 3 are coincide with each other and the mode where each assembly side fixing region 28 is formed with a predetermined spaced interval between the assembly side fixing region 28 and the side edge 3c of the absorbent assembly 3.

The waist elasticized portion G1 also includes the plurality of fusion bonded region rows 26Sa formed at intervals in the article lateral direction Y. More specifically, the plurality of fusion bonded region rows 26Sa are arranged over a range from a position in the vicinity of one outer fixing region 27 to a position in the vicinity of the other outer fixing region 27. The below-waist lower elasticized portion G2 also includes the plurality of fusion bonded region rows 26Sb formed over a range from a position in the vicinity of one outer fixing region 27 to a position in the vicinity of the other outer fixing region 27. Between the pair of assembly side fixing regions 28, no elastic member 24 is arranged, or the elastic members 24 are arranged in a state where processing to prevent exhibition of elastic stretchability, such as cutting, is performed on the elastic members 24.

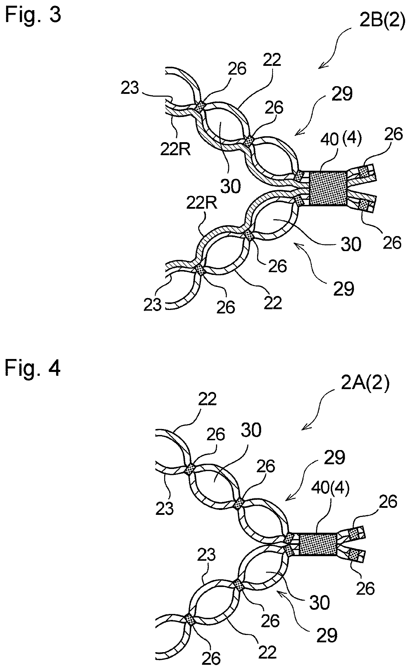

In the waist elasticized portion G1 of the diaper 1 in this embodiment, as shown in Figure 3, the contraction of the elastic members 24, disposed between the outer sheet 22 and the inner sheet 23 in an uncontracted state, causes the sheet 22 and the sheet 23 between the adjacent fusion bonded region rows 26Sa to be deformed to bulge outward. Accordingly, between the adjacent fusion bonded region rows 26Sa, a pleat 29 formed of the sheet 22 or sheets 23, 22R is formed and, at the same time, a hollow portion 30 surrounded by the pleat 29 and the pleat 29 is formed between both sheets 22, 23.

In the diaper 1 of this embodiment, a sheet material which forms the outer sheet 22 of each of the front portion A and the rear portion B is folded back to the skin-facing surface side of the inner sheet 23 along a peripheral edge portion 51 of the waist opening 5. The folded portion 22R covers the skin-facing surface side of the inner sheet 23 of the waist elasticized portion G1 (see Figure 3). Further, the folded portion 22R is joined to the skin-facing surface of the inner sheet 23 and/or the absorbent assembly 3 by an adhesive agent which is entirely or partially applied to the folded portion 22R. Whereas the folded portion 22R is not fusion bonded to the fusion bonded region 26.

In the below-waist lower elasticized portion G2, as shown in Figure 4, the contraction of the elastic members 24, disposed in an uncontracted state between the outer sheet 22 and the inner sheet 23, causes the sheet 22 and the sheet 23 between the adjacent fusion bonded region rows 26Sb to be deformed to bulge outward. Accordingly, the pleat 29 formed of the sheet 22 or the sheet 23 is formed between the adjacent fusion bonded region rows 26Sb and, at the same time, the hollow portion 30 surrounded by the pleat 29 and the pleat 29 is formed between both sheets 22, 23.

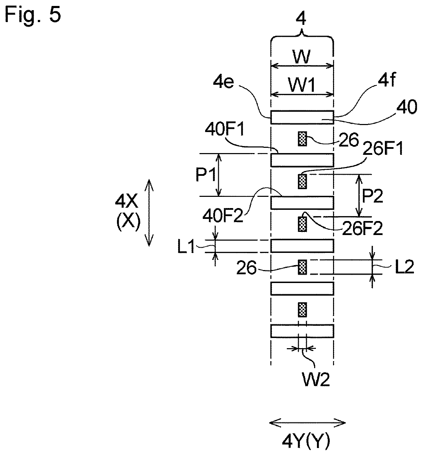

As shown in Figure 5, the side seal 4 includes joined regions 40 where the front portion A and the rear portion B are joined. The side seals 4 in this embodiment are formed such that both sides of the front portion A and both sides of the rear portion B are made to overlap with each other, and the overlapping portions are joined with each other by heating and pressing at a plurality of portions of the side seal 4 arranged along a longitudinal direction 4X of the side seal 4. The side seal 4 in this embodiment includes the plurality of joined regions 40 which are intermittently arranged to form one row along the longitudinal direction 4X of the side seal 4.

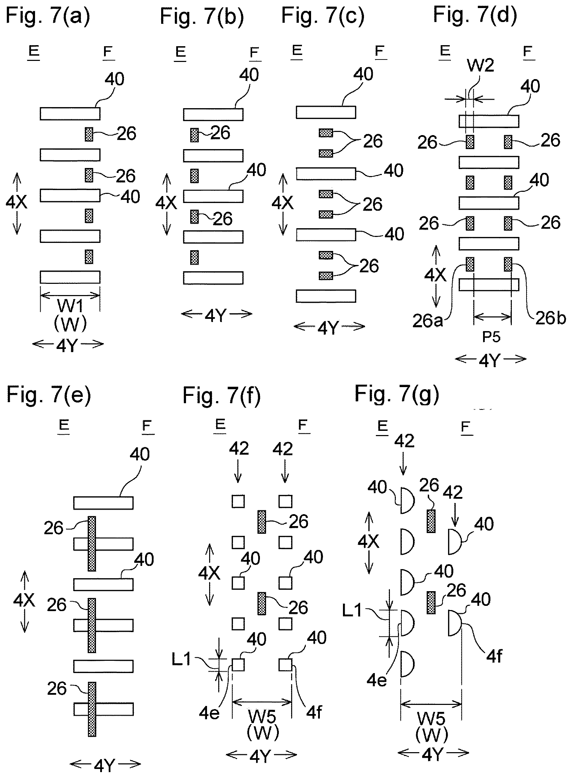

The side seal 4 is a region where the joined regions 40 are present in the width direction of the side seal 4. As shown in Figure 7(f) and Figure 7(g), in the case where the side seal 4 includes the plurality of joined regions 40 arranged in the width direction of the side seal 4, the side seal 4 is a region ranging from an inner end portion 4e of the joined region 40 positioned on the innermost side in the width direction of the side seal 4 to an outer end portion 4f of the joined region 40 positioned on the outermost side in the width direction of the side seal 4.

The longitudinal direction 4X of the side seal 4 is a direction along which the side seal 4 extends, and is usually a direction which extends along the article longitudinal direction X of the pull-on garment. A width direction 4Y of the side seal 4 is a direction orthogonal to the longitudinal direction 4X of the side seal 4, and is usually a direction which extends along the article lateral direction Y of the pull-on garment.

A length W of the side seal 4 in the width direction 4Y (see Figure 5) is a distance between the inner end portion 4e of the joined region 40 positioned on the innermost side in the width direction 4Y of the side seal 4 and the outer end portion 4f of the joined region 40 positioned on the outermost side in the width direction 4Y of the side seal 4. The length W is measured along the width direction 4Y of the side seal 4. As shown in Figure 7(f) or Figure 7(g), the inner end portion 4e of the joined region positioned on the innermost side and the outer end portion 4f of the joined region positioned on the outermost side may be end portions 4e, 4f of different joined regions 40. In this embodiment, the inner side is the side closer to the absorbent assembly 3 in the width direction 4Y of the side seal, and the outer side is the side more remote from the absorbent assembly 3.

Further, the description "inward of the center position" in the width direction of the side seal 4 described later means the side of the joined region 40 closer to the absorbent assembly 3 than the center position of the joined region 40 in the above-mentioned width direction. Further, the description "outward of the center position" in the width direction of the side seal 4 means the side more remote from the wearer's skin than the above-mentioned center position. The center position of the joined region 40 in the above-mentioned width direction is a position of a center line which bisects the length of the joined region 40 in the width direction of the side seal.

The side seal 4 of the diaper 1 of this embodiment includes, as shown in Figure 5, the fusion bonded regions 26 in addition to the above-mentioned joined regions 40. The joined region 40 is a portion where the front portion A and the rear portion B are joined with each other. Whereas the fusion bonded region 26 is a portion where a plurality of sheets which form the front portion A are fusion bonded or a plurality of sheets which form the rear portion B are fusion bonded. The fusion bonded regions 26 of the front portion A are formed by performing sealing on the plurality of sheets which form the front portion A before the sheets which form the rear portion B are made to overlap. The fusion bonded regions 26 of the rear portion B are formed by performing sealing on the plurality of sheets which form the rear portion B before the sheets which form the front portion A are made to overlap. Sealing is processing to form the fusion bonded region where the plurality of sheets are joined by fusion bonding, and not only heat sealing, such as heat embossing, but also ultrasonic sealing, high frequency sealing or other sealing may be used.

In the diaper 1 of this embodiment, a large number of fusion bonded regions 26 are provided not only to the side seals of the outer cover 2 but also to portions of the outer cover 2 other than the side seals and, as shown in Figure 2, some of the large number of fusion bonded regions 26 are present in the side seal 4. However, the outer cover 2 may include the fusion bonded regions 26 only in the side seal 4. The side seal 4 may include only the fusion bonded regions 26 where the plurality of constituent sheets of the front portion A are fusion bonded. The side seal 4 may include only the fusion bonded regions 26 where the plurality of constituent sheets of the rear portion B are fusion bonded. The side seal 4 may include both the fusion bonded regions 26 where the plurality of constituent sheets of the front portion A are fusion bonded, and the fusion bonded regions 26 where the plurality of constituent sheets of the rear portion B are fusion bonded.

In the side seal 4 in this embodiment, as shown in Figure 5, the joined regions 40 are formed intermittently in the longitudinal direction of the side seal 4, and each fusion bonded region 26 is disposed between the joined regions 40 disposed adjacent to each other in the longitudinal direction 4X. More specifically, the joined region 40 and the fusion bonded region 26 are arranged alternately in the longitudinal direction 4X of the side seal 4. The fusion bonded region 26 is not necessarily formed in every space formed between the joined regions 40 present in the side seal 4. A space formed between the joined regions 40 and having the fusion bonded region 26 and a space formed between the joined regions 40 and having no fusion bonded region 26 may be arranged in an appropriate order in the longitudinal direction of the side seal 4. In the mode shown in Figure 7(f), the space formed between the joined regions 40 and having the fusion bonded region 26 and the space formed between the joined regions 40 and having no fusion bonded region 26 are arranged alternately along the longitudinal direction 4X of the side seal 4.

Further, in the side seal 4 in this embodiment, a length W2 of the fusion bonded region 26 in the width direction 4Y of the side seal 4 is shorter than the length W of the side seal 4 in the width direction 4Y of the side seal 4. Further, the entire fusion bonded region 26 falls within the width direction of the side seal 4. In the present invention, the fusion bonded region 26 may partially extend inward from the inner end portion of the side seal 4 in the width direction of the side seal 4. In this case, the length of the portion extending from the side seal 4 is not included in the length W2 of the fusion bonded region, and only the length of a portion of the fusion bonded region which is present in the side seal 4 is assumed as the length W2 of the fusion bonded region.

The diaper 1 of this embodiment can be worn in the same manner as a normal pull-on disposable diaper. At the time of removing the diaper 1 after wear, the diaper 1 can be removed from a wearer by tearing the side seals in the same manner as the normal pull-on disposable diaper.

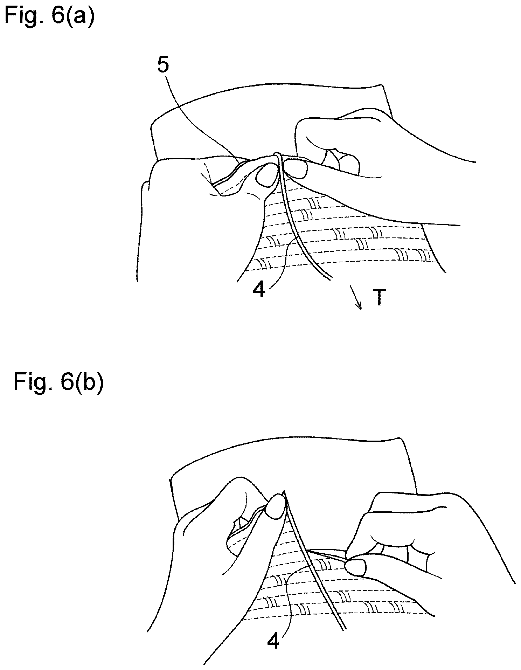

At the time of tearing the side seal 4 of the diaper 1 of this embodiment from the waist opening 5 side, as shown in Figure 6(a), for example, both sides of the side seal 4 are respectively grasped with hands at portions in the vicinity of the waist opening 5, and the side seal 4 is torn toward the leg opening 6 side. A direction toward the leg opening 6 side is a direction T in Figure 6(a). Then, as shown in Figure 6(b), the side seal 4 is torn toward the leg opening 6 side.

According to the findings of the inventors of the present invention, even in the case where the direction along which the side seal 4 is torn is the direction T in Figure 6(a), the following phenomenon occurs at respective portions of the side seal in the longitudinal direction.

On tearing the side seal 4, first, peeling between the front panel 2A and the rear panel 2B starts at the inner end portion of the side seal 4 in the width direction, and constituent fibers of the front panel 2A and the rear panel 2B which are bonded with each other are pulled in directions along which both sheet panels 2A, 2B are peeled. Along the progress of the tearing of the side seal 4 from the inner side toward the outer side in the width direction of the side seal 4, the number of pulled constituent fibers increases. With the increase in the number of pulled constituent fibers, a joining strength at the joined region 40 increases, thus causing the side seal 4 not to be torn easily.

On the other hand, in the side seal 4 of the pull-on garment of the present invention, constituent fibers at the fusion bonded regions 26 are formed into resin compositions so that the form of fibers is not maintained. Accordingly, the number of constituent fibers pulled on tearing is smaller compared with the case of the side seal having no fusion bonded region and hence, a joining strength of the side seal 4 at the time of tearing is reduced compared with the side seal having no fusion bonded region. Therefore, it is possible to easily tear the side seal 4.

The present invention can acquire the above-mentioned advantageous effect of reducing a joining strength of the side seal 4, and the acquisition of the advantageous effect can be confirmed from the results of the following tear test, for example.

First, two nonwoven fabrics having a size of 30 mm × 50 mm are joined by fusion bonding, and two sets of the joined nonwoven fabrics are made to overlap with each other. Then, the two sets of the joined nonwoven fabrics are sealed at one portion by an ultrasonic crimp sealer US-60B made by FUJIIMPULSE to form a joined region. The member formed as described above is assumed as a measurement piece "a". Four nonwoven fabrics having a size of 30 mm × 50 mm are made to overlap with each other, and are sealed at one portion by the crimp sealer to form a joined region. The member formed as described above is assumed as a measurement piece "b". The measurement piece "a" and the measurement piece "b" are test pieces which imitate portions of the side seal. The joined region of each measurement piece "b" has a shape and size identical to the shape and size of the measurement piece "a", and has a rectangular shape elongated in one direction. Further, the length of each fusion bonded region of the measurement piece "a" along the longitudinal direction of the joined region is shorter than the length of the joined region in the longitudinal direction of the joined region. The fusion bonded regions of the measurement piece "a" are formed with the same pattern as the peripheral region S of the side seal 4 shown in Figure 2. As nonwoven fabrics used for the measurement piece "a" and the measurement piece "b", sheet materials used for the outer sheet 22 and the inner sheet 23 described later are used.

Next, the measurement piece "a" and the measurement piece "b" are torn along the longitudinal direction of the joined region at a pulling speed of 300 mm/min, and a tear strength (N) during tearing is measured. A tear strength is measured by using AUTOGRAPH AG-X made by SHIMADZU CORPORATION.

In the above-mentioned measurement method, with the progress of tearing of the measurement piece, that is, with an increase in tearing distance of the measurement piece, a tear strength of either one of the measurement piece "a" and the measurement piece "b" increases and, after the tear strength reaches the maximum value, the tear strength decreases. For example, the maximum value of the measurement piece "a" is 4.3 N, and the maximum value of the measurement piece "b" is 6.2 N. As described above, the maximum value of the tear strength of the measurement piece "a" is a value smaller than the maximum value of the tear strength of the measurement piece "b". The reason of the above is considered as follows. Constituent fibers in the fusion bonded regions of the measurement piece "a" are formed into resin compositions so that the form of fibers is not maintained. Accordingly, the number of constituent fibers pulled on tearing is smaller than that of the measurement piece "b" having no fusion bonded region. The result shows that the joining strength of the measurement piece "a" on tearing is reduced compared with that of the measurement piece "b" having no fusion bonded region.

Further, in the side seal 4 of the pull-on garment of the present invention, the length of the fusion bonded region 26 in the width direction of the side seal 4 is shorter than the length W of the side seal 4 in the width direction of the side seal 4. Accordingly, on tearing the side seal 4, a pulling force can be easily transferred in the longitudinal direction of the side seal 4 and hence, it is possible to easily tear the side seal 4 in the longitudinal direction of the side seal 4.

It is necessary to prevent the side seal 4 of the pull-on garment from being unintentionally torn during wear of the pull-on garment. For example, there may be a case where movement of the wearer applies a force which peels the joined region to the side seal, thus causing the side seal 4 to be torn. According to the diaper 1 of this embodiment, it is possible to prevent unintentional peeling of the side seal 4 during wear. Specifically, a force which peels the joined region 40 is easily transferred through portions formed into resin compositions. However, in the side seal 4 of the pull-on garment of the present invention, the length of the fusion bonded region 26 in the width direction of the side seal 4 is shorter than the length W of the side seal 4 in the width direction of the side seal 4. Accordingly, the side seal 4 includes no portion where the entire range of the side seal 4, including the fusion bonded regions 26, in the width direction of the side seal 4 is formed into a resin composition. Therefore, a required minimum seal strength during wear is ensured to prevent the side seal 4 from being unintentionally torn.

As described above, according to the pull-on garment of the present invention, it is possible to easily realize both a joining strength of the side seal during wear and ease in tearing the side seal at the time of removing the pull-on garment.

Further, the side seals 4 are portions grasped at the time of tearing a diaper, thus being important portions which give an impression of softness of a product and hence, it is preferable that the side seal 4 is not hard. In the side seal 4 of the pull-on garment of the present invention, the length of the fusion bonded region 26 in the width direction of the side seal 4 is shorter than the length W of the side seal 4 in the width direction of the side seal 4. Accordingly, it is possible to suppress that the side seal 4 is excessively cured due to portions formed into resin compositions. At the same time, there are one or more portions which are not formed into resin compositions around the fusion bonded region 26 and hence, such portions form bending points, thus enhancing softness of the entire side seal 4. To the contrary, in the case where the entire side seal in the width direction is formed into a resin composition by the fusion bonded region, the side seal is excessively cured by the fusion bonded region, thus being hardened.

As shown in Figure 5, the arrangement pattern of the joined regions 40 and the fusion bonded regions 26 in the side seal 4 in this embodiment includes the fusion bonded regions 26 at the center of the side seal 4 in the width direction. The center position of the side seal 4 in the width direction and the center positions of the fusion bonded regions 26 in the width direction are coincide with each other. It is preferable that the side seal 4 includes the plurality of fusion bonded regions 26 as described above. In addition to the pattern shown in Figure 5, patterns shown in Figures 7(a) to (g), for example, can be named as the arrangement pattern of the joined regions 40 and the fusion bonded regions 26 in the side seal 4.

As shown in Figure 7(a), the fusion bonded regions 26 may be disposed outward of the center position of the side seal 4 in the width direction. As shown in Figure 7(b), being an embodiment according to the invention, the fusion bonded regions 26 may be disposed inward of the center position of the side seal 4 in the width direction. In Figure 7, an area outward of the center position of the side seal 4 in the width direction is donated by "F", and an area inward of the center position is donated by "E".

It is according to the invention that the fusion bonded regions 26 are, as shown in Figure 7(b), positioned inward of the center position of the side seal 4 in the width direction. In general, at the time of disposing a diaper, the side seal is torn from the inside. Accordingly, with the provision of the fusion bonded regions disposed inward of the center position, constituent fibers at an initial stage of tearing the side seal 4 are formed into resin compositions to eliminate the shape of fibers. With such a configuration, it is possible to prevent an increase in the number of constituent fibers pulled at the time of tearing the side seal 4 and hence, the side seal 4 can be torn more easily.

Only some of the fusion bonded regions 26 may be disposed inward of the center position of the side seal 4 in the width direction. However, it is preferable that all fusion bonded regions 26 are disposed inward of the center position as shown in Figure 7(b).

The fusion bonded regions 26 and the joined regions 40 may be arranged such that the inner side edges of the fusion bonded regions 26 and the inner side edges of the joined regions 40 in the width direction of the side seal 4 or the outer side edges of the fusion bonded regions 26 and the outer side edges of the joined regions 40 in the width direction of the side seal 4 overlap each other in the longitudinal direction of the side seal 4.

The side seal 4 in this embodiment shown in Figure 5 and the side seal 4 shown in Figures 7(a) to (g) includes the plurality of joined regions 40 arranged in one row along the longitudinal direction of the side seal 4, and includes one or more fusion bonded regions 26 at positions in the vicinity of the joined regions 40. It is preferable that the side seal 4 includes the plurality of joined regions 40 intermittently aligned in the longitudinal direction of the side seal 4, and includes one or more fusion bonded regions 26 located between the joined regions 40 as described above. With such a configuration, it is possible to reduce a joining strength of the joined regions 40 so that the side seal 4 can be torn more easily.

From the viewpoint of more easily realizing both ease in tearing the side seal 4 and a joining strength of the side seal 4, the total area of the fusion bonded regions 26 in the side seal 4 is preferably set to 10% or more, and more preferably set to 150% or more of the total area of the joined regions 40. The total area of the fusion bonded regions 26 is preferably set to 700% or less, and more preferably set to 450% or less of the total area of the joined regions 40. Further, the total area of the fusion bonded regions 26 is preferably set to 10% or more and 700% or less, and more preferably set to 150% or more and 450% or less of the total area of the joined regions 40. The total area of the fusion bonded regions 26 is the total of the total area of the fusion bonded regions 26 in the side seal 4 of the front portion A and the total area of the fusion bonded regions 26 in the side seal 4 of the rear portion B.

From the same viewpoint as the above, the area of one fusion bonded region 26 in the side seal 4 is preferably set to 10% or more, and more preferably set to 50% or more of the area of one joined region 40. The area of the one fusion bonded region 26 is preferably set to 300% or less, and more preferably set to 120% or less of the area of the one joined region 40. Further, the area of the one fusion bonded region 26 is preferably set to 10% or more and 300% or less, and more preferably set to 50% or more and 120% or less of the area of the one joined region 40.

In the outer cover 2 in this embodiment, the front panel 2A and the rear panel 2B include the fusion bonded regions 26. The fusion bonded regions 26 shown in Figure 5 and Figures 7(a) to (g) may be the fusion bonded regions 26 of the front portion A, the fusion bonded regions 26 of the rear portion B, or the fusion bonded regions 26 of the front portion A and the rear portion B. As described above, the side seal 4 is formed by joining portions where both sides of the front portion A and both sides of the rear portion B are made to overlap with each other. However, the fusion bonded regions 26 in the side seal 4 of the front panel 2A and the fusion bonded regions 26 in the side seal 4 of the rear panel 2B may or may not overlap with each other.

It is preferable that the side seal 4 of the front portion A and the side seal 4 of the rear portion B include the fusion bonded regions 26. It is also preferable that the fusion bonded regions 26 of the front portion A and the fusion bonded regions 26 of the rear portion B differ in position in the width direction 4Y of the side seal 4.

The fusion bonded regions 26 of the front portion A and the fusion bonded regions 26 of the rear portion B coexist in a state where the fusion bonded regions 26 of the front portion A and the fusion bonded regions 26 of the rear portion B differ in position in the width direction 4Y of the side seal 4. Accordingly, it is possible to suppress that the fusion bonded regions 26 are partially concentrated, thus partially and extremely reducing a joining strength of the side seal 4.

In this embodiment, it is sufficient for the fusion bonded regions 26 of the front portion A and the fusion bonded regions 26 of the rear portion B to be displaced in the longitudinal direction, the width direction, or the longitudinal direction and the width direction of the side seal 4. The fusion bonded regions 26 of the front portion A and the fusion bonded regions 26 of the rear portion B may partially overlap with each other. From the viewpoint of equalizing a joining strength along the entire side seals 4, an area of a portion where the fusion bonded regions 26 in the side seal 4 of the front portion A and the fusion bonded regions 26 in the side seal 4 of the rear portion B overlap with each other is preferably set to 50% or less, and more preferably set to 20% or less of a total area of the fusion bonded regions 26 in the side seal 4 of the front portion A and the fusion bonded regions 26 in the side seal 4 of the rear portion B.

The arrangement pattern shown in Figure 5, Figure 7(a) and Figure 7(b) includes one fusion bonded region 26 between the joined regions 40 disposed adjacent to each other in the longitudinal direction of the side seal 4. The arrangement pattern of the joined regions 40 and the fusion bonded regions 26 in the side seal 4 includes, as shown in Figure 7(c) and Figure 7(d), two fusion bonded regions 26 between the joined regions 40 disposed adjacent to each other in the longitudinal direction of the side seal 4. With such a configuration, it is possible to tear the side seal 4 more easily. As described above, it is preferable that the arrangement pattern includes the plurality of fusion bonded regions 26 between the joined regions 40.

From the viewpoint of more easily realizing both ease in tearing the side seal 4 and a joining strength of the side seal 4, the number of fusion bonded regions 26 disposed between the joined regions 40 disposed adjacent to each other in the longitudinal direction of the side seal 4 is preferably set to 2 or more, and more preferably set to 3 or more, is preferably set to 10 or less, and more preferably set to 5 or less, and is preferably set to 2 or more and 10 or less, and more preferably set to 3 or more and 5 or less.

The arrangement pattern of the joined regions 40 and the fusion bonded regions 26 shown in Figure 7(c) includes, between the joined regions 40 disposed adjacent to each other in the longitudinal direction of the side seal 4, the plurality of fusion bonded regions 26 aligned in the longitudinal direction of the side seal 4. As described above, the plurality of fusion bonded regions 26 may be aligned in the longitudinal direction of the side seal 4.

In the arrangement pattern of the joined regions 40 and the fusion bonded regions 26 shown in Figure 7(d), between each space formed between the joined regions 40 disposed adjacent to each other in the longitudinal direction of the side seal 4, the fusion bonded region 26 disposed on the inner side in the width direction of the side seal 4 and the fusion bonded region 26 disposed on the outer side in the width direction of the side seal 4 are aligned in the width direction of the side seal 4. With such a configuration, the fusion bonded regions 26 are aligned substantially along the direction along which the side seal 4 is torn and hence, the side seal 4 can be torn more easily. As described above, it is preferable that the plurality of fusion bonded regions 26 are aligned in the width direction 4Y of the side seal 4.

Further, from the viewpoint of equalizing a joining strength of the side seal 4 in the width direction of the side seal 4, it is more preferable that the plurality of fusion bonded regions 26 aligned in the width direction of the side seal 4 are aligned at equal intervals in the width direction 4Y of the side seal 4.

As described above, the fusion bonded region 26 is formed by causing the outer sheet 22 and the inner sheet 23 to be fusion bonded with each other. Accordingly, the fusion bonded region 26 has low softness, and is easily hardened. From the viewpoint of softening the side seal 4 to facilitate tearing of the side seal 4, as shown in Figure 7(d), the plurality of fusion bonded regions 26 are arranged in the width direction 4Y of the side seal 4 at an arrangement pitch P5 of the fusion bonded regions 26 in the width direction 4Y of the side seal 4, and the arrangement pitch P5 is preferably two times or more, and more preferably three times or more of the length W2 of the fusion bonded region 26 in the width direction 4Y. The arrangement pitch P5 of the fusion bonded regions 26 in the width direction is a length P5 from the outer end of a fusion bonded region 26a disposed on the innermost side in the width direction to the outer end of another fusion bonded region 26b disposed adjacent to the fusion bonded region 26a in the width direction.

Further, from the viewpoint of averaging a force required for peeling in the width direction of the side seal, it is preferable that the fusion bonded region 26 of the front portion A and the fusion bonded region 26 of the rear portion B, which are disposed at different positions in the width direction 4Y of the side seal 4, are aligned in the width direction of the side seal 4. It is also preferable that the fusion bonded region 26 of the front portion A and the fusion bonded region 26 of the rear portion B are formed in a common space formed between the joined regions disposed adjacent to each other in the longitudinal direction 4X.

For example, in the side seal 4 shown in Figure 7(d), only the fusion bonded regions of the front portion A or only the fusion bonded regions of the rear portion B side may be formed with the mode shown in Figure 7(d). Alternatively, the fusion bonded regions of the front portion A and the fusion bonded regions on the rear portion B side may exist in a mixed manner in the mode shown in Figure 7(d). For example, the fusion bonded regions disposed outward of the center position may be the fusion bonded regions of the front portion A, and the fusion bonded regions disposed inward of the center position may be the fusion bonded regions of the rear portion B. On the other hand, the fusion bonded regions disposed inward of the center position may be the fusion bonded regions of the front portion A, and the fusion bonded regions disposed outward of the center position may be the fusion bonded regions of the rear portion B. Further, the fusion bonded region of the front portion A and the fusion bonded region of the rear portion B may coexist at a plurality of positions in the longitudinal direction such that the fusion bonded region of the front portion A and the fusion bonded region of the rear portion B are alternately positioned.

As shown in Figure 7(e), the fusion bonded region 26 may be disposed to extend over a range between one space formed between the joined regions 40 disposed adjacent to each other in the longitudinal direction 4X of the side seal 4 and another space formed between the joined regions 40 disposed adjacent to each other in the longitudinal direction 4X.

In the arrangement pattern of the joined regions 40 and the fusion bonded regions 26 shown in Figure 7(e), the fusion bonded region 26 extending in the longitudinal direction 4X of the side seal 4 is disposed on the inner side of the side seal 4 in the width direction 4Y, and includes one portion which overlaps with the joined region 40. With such a configuration, it is possible to more easily realize both a joining strength of the side seal and ease in tearing. It is preferable to include a portion where the joined region 40 and the fusion bonded region 26 overlap with each other as described above.

In the side seal 4 in this embodiment, as shown in Figure 5, the plurality of joined regions 40 are arranged in one row in the longitudinal direction 4X of the side seal 4. However, as shown in Figure 7(f) and Figure 7(g), the side seal 4 may include, in the width direction 4Y of the side seal 4, a plurality of joined region rows 42 each of which is formed of the plurality of joined regions 40 arranged in the longitudinal direction 4X of the side seal 4. In the side seal 4, as shown in Figure 7(g), the number of joined regions 40 which form the joined region row 42 may differ for the respective joined region rows 42.

In the case where the side seal 4 includes the plurality of joined region rows 42 as shown in Figure 7(f) and Figure 7(g), a length W5 from the inner side edge of the joined region row 42 positioned on the innermost side in the width direction 4Y of the side seal 4 to the outer side edge of the joined region row 42 positioned on the outermost side in the width direction 4Y of the side seal 4 is also referred to as a width W5 of the joined region row 42. The width W5 of the joined region row 42 is longer than the length of the fusion bonded region 26 in the width direction 4Y of the side seal 4. Further, it is preferable that the width W5 of the joined region row 42 is longer than a length L1 of the joined region 40 in the longitudinal direction 4X of the side seal 4.

As shown in Figure 5, Figures 7(a) to (e), the joined region 40 may have a shape elongated in the width direction 4Y of the side seal 4, or may have a shape elongated in the longitudinal direction 4X of the side seal 4. From the viewpoint of more easily realizing both a joining strength of the side seal and ease in tearing, it is preferable that the length L1 of the joined region 40 in the longitudinal direction of the side seal 4 (see Figure 5) is shorter than the length of the side seal 4 in the width direction (see Figure 5).

From the viewpoint of acquiring the above-mentioned advantageous effects with more certainty, the length L1 of the joined region 40 in the longitudinal direction of the side seal 4 (see Figure 5) is preferably set to 5% or more, and more preferably set to 15% or more of the length W of the side seal 4 in the width direction of the side seal 4 (see Figure 5). The length L1 is preferably set to 100% or less, and more preferably set to 50% or less of the length W. Further, the length L1 is preferably set to 5% or more and 100% or less, and more preferably set to 15% or more and 50% or less of the length W.

From the same viewpoint, a length L2 of the fusion bonded region 26 in the longitudinal direction of the side seal 4 (see Figure 5) is preferably set to 10% or more, and more preferably set to 50% or more of the length W2 of the side seal 4 in the width direction of the side seal 4 (see Figure 5). The length L2 is preferably set to 800% or less, and more preferably set to 400% or less of the length W2. Further, the length L2 is preferably set to 10% or more and 800% or less, and more preferably set to 50% or more and 400% or less of the length W2.

From the viewpoint of easily realizing both a joining strength of the side seal and ease in tearing the side seal, it is preferable that the arrangement of the joined regions 40 and the fusion bonded regions 26 in the side seal 4 falls within the following range.

It is preferable that an arrangement pitch P1 of the joined regions 40 in the longitudinal direction of the side seal 4 (see Figure 5) is longer than the length W of the side seal 4 in the width direction of the side seal 4 or a length W1 of the joined region 40 in the width direction of the side seal 4 (see Figure 5). As shown in Figure 5, the arrangement pitch P1 of the joined regions 40 in the longitudinal direction of the side seal 4 is a length P1 from an upper end 40F1 of one joined region 40 in the longitudinal direction of the side seal 4 to an upper end 40F2 of another joined region 40 disposed adjacent to the one joined region 40 in the longitudinal direction of the side seal 4.

The arrangement pitch P1 of the joined regions 40 in the longitudinal direction of the side seal 4 (see Figure 5) is preferably set to 50% or more, and more preferably set to 60% or more of the length W of the side seal 4 in the width direction of the side seal 4 or the length W1 of the joined region 40 in the width direction of the side seal 4 (see Figure 5). The arrangement pitch P1 is preferably set to 200% or less, and more preferably set to 160% or less of the length W or the length W1. Further, the arrangement pitch P1 is preferably set to 50% or more and 200% or less, and more preferably set to 60% or more and 160% or less of the length W or the length W1.

The arrangement pitch P1 of the joined regions 40 in the longitudinal direction of the side seal 4 (see Figure 5) is preferably set to 100% or more, and more preferably set to 300% or more of the length L1 of the joined region 40 in the longitudinal direction of the side seal 4 (see Figure 5). The arrangement pitch P1 is preferably set to 1000% or less, and more preferably set to 700% or less of the length L1. Further, the arrangement pitch P1 is preferably set to 100% or more and 1000% or less, and more preferably set to 300% or more and 700% or less of the length L 1.

An arrangement pitch P2 of the fusion bonded regions 26 in the longitudinal direction of the side seal 4 (see Figure 5) is preferably set to 10% or more, and more preferably set to 100% or more of the length L2 of the fusion bonded region 26 in the longitudinal direction of the side seal 4 (see Figure 5). The arrangement pitch P2 is preferably set to 600% or less, and more preferably set to 500% or less of the length L2. Further, the arrangement pitch P2 is preferably set to 10% or more and 600% or less, and more preferably set to 100% or more and 500% or less of the length L2. The arrangement pitch P2 of the fusion bonded regions 26 in the longitudinal direction of the side seal 4 is, as shown in Figure 5, a length P2 from an upper end 26F1 of one fusion bonded region in the longitudinal direction of the side seal 4 to an upper end 26F2 of another fusion bonded region 26 disposed adjacent to the one fusion bonded region 26 in the longitudinal direction of the side seal 4.

The arrangement pitch P2 of the fusion bonded regions 26 in the longitudinal direction of the side seal 4 (see Figure 5) is preferably set to 300% or more, and more preferably set to 400% or more of the length W2 of the fusion bonded region 26 in the width direction of the side seal 4 (see Figure 5). The arrangement pitch P2 is preferably set to 2000% or less, and more preferably set to 1400% or less of the length W2. Further, the arrangement pitch P2 is preferably set to 300% or more and 2000% or less, and more preferably set to 400% or more and 1400% or less of the length W2.

From the viewpoint of disposing one fusion bonded region 26 for one joined region 40 to facilitate tearing of the side seal 4, it is preferable that the arrangement pitch P2 of the fusion bonded regions 26 in the longitudinal direction of the side seal 4 (see Figure 5) is shorter than the arrangement pitch P1 of the joined regions 40 in the longitudinal direction of the side seal 4 (see Figure 5). The arrangement pitch P2 of the fusion bonded regions 26 in the longitudinal direction of the side seal 4 (see Figure 5) is preferably set to 10% or more, and more preferably set to 50% or more of the arrangement pitch P1 of the joined regions 40 in the longitudinal direction of the side seal 4 (see Figure 5). The arrangement pitch P2 is preferably set to 100% or less, and more preferably set to 90% or less of the arrangement pitch P1. Further, the arrangement pitch P2 is preferably set to 10% or more and 100% or less, and more preferably set to 50% or more and 90% or less of the arrangement pitch P1.

As shown in Figure 2, the side seal 4 in this embodiment includes the fusion bonded regions 26 in the entire region of the side seal 4 in the longitudinal direction 4X. However, the side seal 4 may partially include the fusion bonded regions 26 in the longitudinal direction of the side seal 4.

From the viewpoint of tearing the side seal 4 more easily, it is preferable that the side seal 4 includes the fusion bonded regions in at least one region on the waist opening 5 side out of two regions defined by dividing the entire side seal 4 in the longitudinal direction 4X into two regions, more preferably out of three regions defined by dividing the entire side seal 4 in the longitudinal direction 4X into three regions.

From the same viewpoint as the above, the side seal 4 includes the fusion bonded regions 26 in a region having a range of preferably 50 mm, and more preferably 30 mm in the longitudinal direction 4X of the side seal 4 from the end portion of the side seal 4 on the waist opening 5 side.

As described above, in the waist elasticized portion G1 and the below-waist lower elasticized portion G2, the elastic members 24 of the diaper 1 of this embodiment extend in the article lateral direction Y while passing through spaces formed between the fusion bonded regions 26 of the respective fusion bonded region rows 26Sa, the fusion bonded regions 26 being disposed adjacently in the article longitudinal direction X. That is, the fusion bonded regions 26 and the elastic members 24 do not overlap with each other in the waist elasticized portion G1 and the below-waist lower elasticized portion G2.

In the diaper 1 of this embodiment, the above-mentioned pleats 29 are formed as shown in Figure 3 and Figure 4 so that the outer cover 2 is soft whereby the diaper 1 has a good wearing feeling. Further, each hollow portion 30 between the pleats 29, 29 formed on the outer surface or the inner surface side of the diaper 1 function as ventilation passages and hence, moisture in the diaper 1 can be easily released to the outside so that stuffiness does not easily occur. It is preferable that such pleats 29 are formed in the waist elasticized portion G1 and/or the below-waist lower elasticized portion G2 at least in a natural state. It is preferable that spaces formed between the pleat 29 and the pleat 29 are maintained also in a state where the article is worn.

From the viewpoint of ensuring air permeability of the pleats 29 formed on the outer cover 2, and facilitating tearing of the side seal 4, it is preferable that the elastic member 24 is disposed in a space formed between the fusion bonded regions 26 disposed adjacent to each other in the article longitudinal direction X.

In the diaper 1, the fusion bonded regions 26 are formed in the front panel 2A and the rear panel 2B at different intervals in the article lateral direction Y. As shown in Figure 2, in the diaper 1, an arrangement pitch P7 of the fusion bonded regions 26 in the article lateral direction Y in peripheral regions S of the side seals 4, that is, in a pair of outer regions S, S positioned outward in the article lateral direction Y of the diaper 1 is shorter than that in a center region M positioned between the outer regions S, S. With such a configuration, in the outer region S, the width of the pleats 29, formed on the outer cover 2, in the article lateral direction Y is shorter than that in the center region M, and the number of fusion bonded regions 26 is larger than that in the center region M. As a result, the outer regions S are not easily stretched in the article lateral direction Y compared with the center region M and hence, lateral tearing does not easily occur in the outer region S at the time of tearing the side seal 4. As described above, in the flat-out, uncontracted state of the diaper 1, it is preferable that the arrangement pitch of the fusion bonded regions 26 in the article lateral direction Y is shorter in the peripheral regions of the side seal 4 than in other portions.

From the viewpoint of more effectively suppressing the lateral tearing in the outer regions S, the arrangement pitch P7 of the fusion bonded regions 26 in the article lateral direction Y in the outer region S (see Figure 2) is preferably set to 100% or less, and more preferably set to 50% or less of an arrangement pitch P8 of the fusion bonded regions 26 in the article lateral direction Y in the center region M (see Figure 2). The arrangement pitch P7, P8 is a length P7, P8 from the outer end of one fusion bonded region 26 to the outer end of another fusion bonded region 26 disposed adjacent to the one fusion bonded region 26 in the article lateral direction Y in the outer region S or the center region M.

From the same viewpoint as the above, in the flat-out, uncontracted state of the diaper 1, it is preferable that the arrangement pitch P7 of the fusion bonded regions 26 in the article lateral direction Y in the outer region S is equal to or shorter than the arrangement pitch of the fusion bonded regions 26 in the article lateral direction Y in other portions. The outer region S preferably falls within a range of 50 mm, and more preferably falls within a range of 30 mm from the outer end portion of the diaper 1 in the article lateral direction Y toward the inside in the article lateral direction Y. The arrangement pitch P7 of the fusion bonded regions 26 in the article lateral direction Y in the outer region S is preferably set to 100% or less, and more preferably set to 50% or less of the arrangement pitch of the fusion bonded regions 26 in other portions.

Shapes of the joined region 40 and the fusion bonded region 26 as viewed in a plan view are not particularly limited. Each of the joined region 40 and the fusion bonded region 26 may have a desired shape, including a quadrangular shape, such as a rectangular shape and a square shape, a circular shape, an elliptical shape, a semicircular shape and the like.

The fusion bonded region 26 of the diaper 1 of this embodiment is formed by a fusion bonded region forming step where the outer sheet 22 and the inner sheet 23 are made to overlap with each other, and heat embossing is performed on the laminated portion.

For the fusion bonded region forming step, a heat embossing apparatus may be used which includes an embossing roller and an anvil roller, for example, the outer peripheral surface of the embossing roller having fusion bonding projections or the like where the distal end surface of the fusion bonding projection has a shape substantially equal to the profile of the fusion bonded region 26.

In the diaper 1 of this embodiment, the fusion bonded regions 26 are formed by performing the fusion bonded region forming step on the outer sheet 22 and the inner sheet 23 which are made to overlap with each other with the elastic members 24 interposed therebetween.

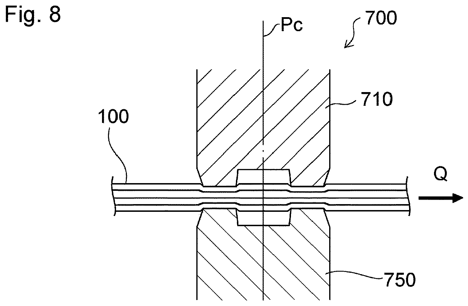

As a method for forming the side seal 4, heat-embossing, ultrasonic embossing, high frequency embossing or the like is used. An apparatus 700 shown in Figure 8 is used for forming the side seals 4 of the diaper 1. As shown in Figure 8, the apparatus 700 includes a seal block 710 and an anvil block 750, and forms the side seal 4 by performing heat-embossing on a continuous body 100 of the diaper 1.

The heating temperature of the seal block 710 and the heating temperature of the anvil block 750 are controlled by a control unit (not shown in the drawing) which the apparatus 700 includes. The sealing surface of the seal block 710 is provided with projections (not shown in the drawing) formed to conform with the shape of the side seal 4 shown in Figure 5, thus forming the pattern of a sealing pattern on a side seal of a diaper.

As shown in Figure 8, the apparatus 700 causes the continuous body 100 of the continuous diapers 1 to pass through between the seal block 710 and the anvil block 750 having a flat surface, and performs embossing at a position of a portion where the side of the front portion A and the side of the rear portion B are made to overlap with each other, thus forming the side seal 4. The seal block 710 includes the projections for forming the joined regions 40. The projections are formed such that a direction orthogonal to a flowing direction Q of the continuous body 100 of the continuous diapers 1 corresponds to the longitudinal direction of the side seal 4 shown in Figure 5. By performing embossing on the continuous body 100 of the diapers 1 between the seal block 710 and the anvil block 750, a pair of sealed portions is formed. This pair of sealed portions form the side seals 4, 4 of the diaper 1 after the continuous body 100 of the diapers 1 are cut at a cut position Pc in a cutting step.

The above-mentioned diaper 1 will be further described. The absorbent assembly 3 includes the liquid permeable topsheet 31, a sparingly liquid permeable backsheet 32, and an absorbent member 33 disposed between the topsheet 31 and the backsheet 32. Sparingly liquid permeable includes liquid impermeable and sparingly liquid permeable. Further, a pair of leak-proof cuff forming sheets 34, 34 are disposed at both sides of the absorbent assembly 3. The leak-proof cuffs forming sheet 34 includes, at a position in the vicinity of the end edge of the leak-proof cuffs forming sheet 34 closer to the article longitudinal direction center line CL, an elastic member 35 for forming leak-proof cuffs. During wear of the diaper, a portion having a predetermined width from the end edge of the leak-proof cuffs forming sheet 34 stands upright to be separated from the topsheet 31 due to a contraction force of the elastic member 35, thus forming leak-proof cuffs.

Materials for forming respective portions of the diaper 1 will be described. For each of the topsheet 31, the backsheet 32, the absorbent member 33, the leak-proof cuffs forming sheet 34 and the like, a material substantially equal to a material which is conventionally used for forming an absorbent article of this kind may be used without any limitation. For example, a liquid permeable nonwoven fabric, a perforated film, a layered body of the liquid permeable nonwoven fabric and the perforated film or the like may be used for the topsheet 31. A resin film, a layered body of the resin film and a nonwoven fabric or the like may be used for the backsheet 32. For the absorbent member 33, a material may be used which is obtained by wrapping, by a core-wrap sheet made of a tissue paper, a water-permeable nonwoven fabric or the like, an absorbent core made of a fiber aggregate of a fiber material, such as pulp, or an absorbent core where the above-mentioned absorbent core is caused to carry superabsorbent polymer. An elastic film, a water-repellent nonwoven fabric, a woven fabric, or laminated sheet of these fabrics may be used for the leak-proof cuffs forming sheet 34.

As a sheet material for forming the outer sheet 22 and the inner sheet 23, any of various sheet materials which are conventionally used for an article of this kind may be used without any limitation. However, it is preferable to use a nonwoven fabric. Particularly, from the viewpoint of softness and the like, it is preferable to use a single layered nonwoven fabric or a two or more layered laminated nonwoven fabric made of an air-through nonwoven fabric, a heat-rolled nonwoven fabric, a spunlace nonwoven fabric, a spun-bonded nonwoven fabric, a melt-blown nonwoven fabric and the like. It is also possible to use a sheet obtained by forming these nonwoven fabrics and a film into an integral body. An outer sheet made of a nonwoven fabric, a film or the like may also be disposed on the non-skin-facing surface side of the backsheet 32 at the crotch portion C.

As a material for forming the elastic member 24, 35, any of various known elastic materials used for absorbent articles, such as disposable diapers and sanitary napkins, may be used without any limitation. A material for forming the elastic material may be synthetic rubber, such as styrene-butadiene, butadiene, isoprene, or neoprene, natural rubber, EVA, elastic polyolefin, polyurethane or the like, for example. The preferred mode of the elastic member to be used may be a filamentous elastic member (thread rubber or the like) or a string-shaped elastic member (flat rubber or the like) having a rectangular shape, a square shape, a circular shape, a polygonal shape or the like in cross section, a filamentous elastic member of a multi-filament type or the like.

The present invention has been described heretofore based on the preferred embodiment of the present invention. However, the present invention is not limited to the above-mentioned embodiment, and may be appropriately modified.

For example, the outer cover 2 of the front portion A or the rear portion B may be configured such that, as in the case of the above-mentioned diaper 1, only the sheet material which forms the outer sheet is folded back at the peripheral edge portion of the waist opening. Alternatively, the sheet material which forms the outer sheet and the sheet material which forms the inner sheet may be integrally folded back, or neither of the outer sheet nor the inner sheet has the folded portion. Further, the folded portion of the outer sheet or the inner sheet may be joined to an opposing surface with a mode different from that of the above-mentioned diaper 1. For example, the folded portion of the outer sheet or the inner sheet may be joined to the entire opposing surface.

### Industrial Applicability

According to the present invention, it is possible to provide the pull-on garment which realizes both a joining strength of the side seal during wear and ease in tearing the side seal at the time of removing.

## Claims

1. A pull-on disposable diaper comprising: a front portion (A) disposed on a front side of a wearer during wear and a rear portion (B) disposed on a rear side of the wearer during wear, both sides of the front portion (A) and both sides of the rear portion (B) being joined with each other to form a pair of side seals (4), a waist opening (5), and a pair of leg openings (6), wherein
the side seals (4) include a joined region (40) where the front portion (A) and the rear portion (B) are joined with each other, and a fusion bonded region (26) where at least either one of a plurality of constituent sheets for the front portion (A) and a plurality of constituent sheets for the rear portion (B) are fusion bonded, and
a length (W2) of the fusion bonded region (26) in a width direction (4Y) of the side seal (4) is shorter than a length (W) of the side seal (4) in the width direction (4Y) of the side seal (4),
wherein the fusion bonded region (26) is positioned inward of a center position of the side seal (4) in the width direction (4Y) of the side seal (4), and
wherein an arrangement pitch (P1) of the joined regions (40) in the longitudinal direction (4X) of the side seal (4) is longer than a length of the joined region (40) in the width direction (4Y) of the side seal (4).

2. The pull-on disposable diaper according to claim 1, wherein the side seals (4) include a plurality of the joined regions (40) arranged intermittently in a longitudinal direction of the side seal (4), and one or more fusion bonded regions (26) located between the joined regions (40).

3. The pull-on disposable diaper according to claim 1 or 2, wherein the side seals (4) include the fusion bonded region (26) on the front portion (A) and on the rear portion (B), and
the fusion bonded region (26) of the front portion (A) and the fusion bonded region (26) of the rear portion (B) are disposed at different positions in the width direction (4Y) of the side seal (4).

4. The pull-on disposable diaper according to claim 3, wherein the fusion bonded region (26) of the front portion (A) and the fusion bonded region (26) of the rear portion (B) are aligned in the width direction (4Y) of the side seal (4), the fusion bonded region (26) of the front portion (A) and the fusion bonded region (26) of the rear portion (B) being disposed at different positions in the width direction (4Y) of the side seal (4).

5. The pull-on disposable diaper according to any one of claims 1 to 4, wherein the side seals (4) include a plurality of the joined regions (40) arranged intermittently in the longitudinal direction (4X) of the side seals (4), and a plurality of the fusion bonded regions (26) in a common space formed between the joined regions (40) disposed adjacent to each other in the longitudinal direction (4X).

6. The pull-on disposable diaper according to claim 4, wherein the fusion bonded region (26) of the front portion (A) and the fusion bonded region (26) of the rear portion (B) are formed in a common space formed between the joined regions (40) disposed adjacent to each other in the longitudinal direction (4X) of the side seal (4).

7. The pull-on disposable diaper according to claim 5 or 6, wherein a plurality of the fusion bonded regions (26) are aligned in a width direction (4Y) of the side seal (4).

8. The pull-on disposable diaper according to any one of claims 5 to 7, wherein the plurality of the fusion bonded regions (26) are aligned at equal intervals in the width direction (4Y) of the side seal (4).

9. The pull-on disposable diaper according to claim 8, wherein an arrangement pitch (P5) of the fusion bonded regions (26) in the width direction (4Y) of the side seal (4) is two times or more of a length of the fusion bonded region (26) in the width direction (4Y).

10. The pull-on disposable diaper according to any one of claims 1 to 10, wherein the pull-on disposable diaper includes a portion where the joined region and the fusion bonded region overlap with each other.

11. The pull-on disposable diaper according to any one of claims 1 to 10, wherein a length of the joined region (40) in the longitudinal direction (4X) of the side seal (4) is shorter than the length of the joined region (40) in the width direction (4Y) of the side seal (4).

12. The pull-on disposable diaper according to any one of claims 1 to 11, wherein the side seals include the fusion bonded region in at least a region on a waist opening side out of two regions defined by dividing an entire side seal in the longitudinal direction into two regions.

13. The pull-on disposable diaper according to any one of claims 1 to 12, wherein an arrangement pitch (P5) of the fusion bonded regions (26) in the longitudinal direction (4X) of the side seal (4) is shorter than the arrangement pitch (P1) of the joined regions (4) in the longitudinal direction (4X) of the side seal (4).

14. The pull-on disposable diaper according to any one of claims 1 to 13, wherein, in a flat-out, uncontracted state of the pull-on disposable diaper, an arrangement pitch (P5) of the fusion bonded regions (26) in the width direction (Y) of the pull-on disposable diaper is shorter in a periphery of the side seal (4) than in a portion other than the periphery of the side seal (4).

15. The pull-on disposable diaper according to claim 14, wherein, in a flat-out, uncontracted state of the pull-on disposable diaper, an arrangement pitch (P5) of the fusion bonded regions (26) in the width direction (Y) of the pull-on disposable diaper is shorter in a region which falls within a range of 50 mm from an outer end portion of the pull-on disposable diaper in the width direction (Y) toward an inside in the width direction (Y) of the pull-on disposable diaper than in a portion other than the range.

## Patentansprüche

1. Wegwerfhöschenwindel, die aufweist: einen vorderen Abschnitt (A), der während des Tragens an einer Vorderseite eines Trägers angeordnet ist, und einen hinteren Abschnitt (B), der während des Tragens an einer Rückseite des Trägers angeordnet ist, wobei beide Seiten des vorderen Abschnitts (A) und beide Seiten des hinteren Abschnitts (B) miteinander verbunden sind, um ein Paar von Seitenverschlüssen (4), eine Taillenöffnung (5) und ein Paar von Beinöffnungen (6) zu bilden, wobei
die Seitendichtungen (4) einen Verbindungsbereich (40), in dem der vordere Abschnitt (A) und der hintere Abschnitt (B) miteinander verbunden sind, und einen Schmelzverbindungsbereich (26) aufweisen, in dem mindestens eine von mehreren Elementarlagen für den vorderen Abschnitt (A) und von mehreren Elementarlagen für den hinteren Abschnitt (B) schmelzverbunden ist, und
eine Länge (W2) des Schmelzverbindungsbereichs (26) in einer Breitenrichtung (4Y) der Seitendichtung (4) kürzer ist als eine Länge (W) der Seitendichtung (4) in der Breitenrichtung (4Y) der Seitendichtung (4),
wobei der Schmelzverbindungsbereich (26) innerhalb einer Mittelposition der Seitendichtung (4) in der Breitenrichtung (4Y) der Seitendichtung (4) positioniert ist, und wobei ein Anordnungsabstand (P1) der Verbindungsbereiche (40) in der Längsrichtung (4X) der Seitendichtung (4) länger ist als eine Länge des Verbindungsbereichs (40) in der Breitenrichtung (4Y) der Seitendichtung (4).

2. Wegwerfhöschenwindel nach Anspruch 1, wobei die Seitendichtungen (4) mehrere in Längsrichtung der Seitendichtung (4) intermittierend angeordneten Verbindungsbereiche (40) und einen oder mehrere zwischen den Verbindungsbereichen (40) angeordnete Schmelzverbindungsbereiche (26) aufweisen.

3. Wegwerfhöschenwindel nach Anspruch 1 oder 2, wobei die Seitendichtungen (4) den Schmelzverbindungsbereich (26) am vorderen Abschnitt (A) und am hinteren Abschnitt (B) aufweisen, und
der Schmelzverbindungsbereich (26) des vorderen Abschnitts (A) und der Schmelzverbindungsbereich (26) des hinteren Abschnitts (B) an unterschiedlichen Positionen in der Breitenrichtung (4Y) der Seitendichtung (4) angeordnet sind.

4. Wegwerfhöschenwindel nach Anspruch 3, wobei der Schmelzverbindungsbereich (26) des vorderen Abschnitts (A) und der Schmelzverbindungsbereich (26) des hinteren Abschnitts (B) in der Breitenrichtung (4Y) der Seitendichtung (4) ausgerichtet sind, wobei der Schmelzverbindungsbereich (26) des vorderen Abschnitts (A) und der Schmelzverbindungsbereich (26) des hinteren Abschnitts (B) an unterschiedlichen Positionen in der Breitenrichtung (4Y) der Seitendichtung (4) angeordnet sind.

5. Wegwerfhöschenwindel nach einem der Ansprüche 1 bis 4, wobei die Seitendichtungen (4) mehrere Verbindungsbereiche (40), die in Längsrichtung (4X) der Seitendichtungen (4) intermittierend angeordnet sind, und mehrere Schmelzverbindungsbereiche (26) in einem gemeinsamen Raum aufweisen, der zwischen den in der Längsrichtung (4X) benachbart angeordneten Verbindungsbereichen (40) ausgebildet ist.

6. Wegwerfhöschenwindel nach Anspruch 4, wobei der Schmelzverbindungsbereich (26) des vorderen Abschnitts (A) und der Schmelzverbindungsbereich (26) des hinteren Abschnitts (B) in einem gemeinsamen Raum ausgebildet sind, der zwischen den in der Längsrichtung (4X) der Seitendichtung (4) benachbart angeordneten Verbindungsbereichen (40) ausgebildet ist.

7. Wegwerfhöschenwindel nach Anspruch 5 oder 6, wobei mehrere Schmelzverbindungsbereiche (26) in einer Breitenrichtung (4Y) der Seitendichtung (4) ausgerichtet sind.

8. Wegwerfhöschenwindel nach einem der Ansprüche 5 bis 7, wobei die mehreren Schmelzverbindungsbereiche (26) in gleichen Abständen in der Breitenrichtung (4Y) der Seitendichtung (4) ausgerichtet sind.

9. Wegwerfhöschenwindel nach Anspruch 8, wobei ein Anordnungsabstand (P5) der Schmelzverbindungsbereiche (26) in der Breitenrichtung (4Y) der Seitendichtung (4) das Zweifache oder mehr einer Länge des Schmelzverbindungsbereichs (26) in der Breitenrichtung (4Y) beträgt.

10. Wegwerfhöschenwindel nach einem der Ansprüche 1 bis 10, wobei die Wegwerfhöschenwindel einen Abschnitt aufweist, in dem sich der Verbindungsbereich und der Schmelzverbindungsbereich überlappen.

11. Wegwerfhöschenwindel nach einem der Ansprüche 1 bis 10, wobei eine Länge des Verbindungsbereichs (40) in der Längsrichtung (4X) der Seitendichtung (4) kürzer ist als die Länge des Verbindungsbereichs (40) in der Breitenrichtung (4Y) der Seitendichtung (4).

12. Wegwerfhöschenwindel nach einem der Ansprüche 1 bis 11, wobei die Seitendichtungen den Schmelzverbindungsbereich in mindestens einem Bereich auf einer Taillenöffnungsseite von zwei Bereichen aufweisen, die durch Unterteilung einer gesamten Seitendichtung in der Längsrichtung in zwei Bereiche definiert sind.

13. Wegwerfhöschenwindel nach einem der Ansprüche 1 bis 12, wobei ein Anordnungsabstand (P5) der Schmelzverbindungsbereiche (26) in der Längsrichtung (4X) der Seitendichtung (4) kürzer ist als der Anordnungsabstand (P1) der Verbindungsbereiche (4) in der Längsrichtung (4X) der Seitendichtung (4).

14. Wegwerfhöschenwindel nach einem der Ansprüche 1 bis 13, wobei in einem flach ausgezogenen, nicht zusammengezogenen Zustand der Wegwerfhöschenwindel ein Anordnungsabstand (P5) der Schmelzverbindungsbereiche (26) in der Breitenrichtung (Y) der Wegwerfhöschenwindel in einem Umfang der Seitendichtung (4) kürzer ist als in einem anderen Abschnitt als dem Umfang der Seitendichtung (4).

15. Wegwerfhöschenwindel nach Anspruch 14, wobei in einem flach ausgezogenen, nicht zusammengezogenen Zustand der Wegwerfhöschenwindel ein Anordnungsabstand (P5) der Schmelzverbindungsbereiche (26) in der Breitenrichtung (Y) der Wegwerfhöschenwindel in einem Bereich, der innerhalb eines Bereichs von 50 mm von einem äußeren Endabschnitt der Wegwerfhöschenwindel in der Breitenrichtung (Y) zu einer Innenseite in der Breitenrichtung (Y) der Wegwerfhöschenwindel fällt, kürzer ist als in einem anderen Abschnitt als dem Bereich.

## Revendications

1. Couche-culotte jetable, comprenant : une partie avant (A) disposée sur le côté avant d'un porteur pendant le port et une partie arrière (B) disposée sur le côté arrière du porteur pendant le port, les deux côtés de la partie avant (A) et les deux côtés de la partie arrière (B) étant raccordés les uns aux autres de manière à former une paire de j oints d'étanchéité latéraux (4), une ouverture de taille (5), et une paire d'ouvertures de jambe (6), où
les joints d'étanchéité latéraux (4) comprennent une zone de raccordement (40) où la partie avant (A) et la partie arrière (B) sont raccordées l'une à l'autre, et une zone de liaison par fusion (26) où au moins une feuille d'une pluralité de feuilles constitutives de la partie avant (A) et d'une pluralité de feuilles constitutives de la partie arrière (B) sont liées par fusion, et
une longueur (W2) de la zone de liaison par fusion (26) dans le sens de la largeur (4Y) du joint d'étanchéité latéral (4) est inférieure à une longueur (W) du joint d'étanchéité latéral (4) dans le sens de la largeur (4Y) du joint d'étanchéité latéral (4),
où la zone de liaison par fusion (26) est présentée à l'intérieur d'un emplacement central du j oint d'étanchéité latéral (4) dans le sens de la largeur (4Y) du j oint d'étanchéité latéral (4), et
où un pas d'agencement (P1) des zones de raccordement (40) dans le sens de la longueur (4X) du joint d'étanchéité latéral (4) est supérieur à une longueur de la zone de raccordement (40) dans le sens de la largeur (4Y) du joint d'étanchéité latéral (4).

2. Couche-culotte jetable selon la revendication 1, où les joints d'étanchéité latéraux (4) comprennent une pluralité de zones de raccordement (40) disposées de manière discontinue dans le sens de la longueur du joint d'étanchéité latéral (4), et une ou plusieurs zones de liaison par fusion (26) situées entre les zones de raccordement (40).

3. Couche-culotte jetable selon la revendication 1 ou la revendication 2, où les joints d'étanchéité latéraux (4) comprennent la zone de liaison par fusion (26) sur la partie avant (A) et sur la partie arrière (B), et
la zone de liaison par fusion (26) de la partie avant (A) et la zone de liaison par fusion (26) de la partie arrière (B) sont présentées à des emplacements différents dans le sens de la largeur (4Y) du joint d'étanchéité latéral (4).

4. Couche-culotte jetable selon la revendication 3, où la zone de liaison par fusion (26) de la partie avant (A) et la zone de liaison par fusion (26) de la partie arrière (B) sont alignées dans le sens de la largeur (4Y) du joint d'étanchéité latéral (4), la zone de liaison par fusion (26) de la partie avant (A) et la zone de liaison par fusion (26) de la partie arrière (B) étant présentées à des emplacements différents dans le sens de la largeur (4Y) du joint d'étanchéité latéral (4).

5. Couche-culotte jetable selon l'une des revendications 1 à 4, où les joints d'étanchéité latéraux (4) comprennent une pluralité de zones de raccordement (40) disposées de manière discontinue dans le sens de la longueur (4X) des joints d'étanchéité latéraux (4), et une pluralité de zones de liaison par fusion (26) dans un espace commun formé entre les zones de raccordement (40) disposées de manière adjacente dans le sens de la longueur (4X).

6. Couche-culotte jetable selon la revendication 4, où la zone de liaison par fusion (26) de la partie avant (A) et la zone de liaison par fusion (26) de la partie arrière (B) sont formées dans un espace commun formé entre les zones de raccordement (40) disposées de manière adjacente dans le sens de la longueur (4X) du joint d'étanchéité latéral (4).

7. Couche-culotte jetable selon la revendication 5 ou la revendication 6, où une pluralité de zones de liaison par fusion (26) sont alignées dans le sens de la largeur (4Y) du joint d'étanchéité latéral (4).

8. Couche-culotte jetable selon l'une des revendications 5 à 7, où la pluralité de zones de liaison par fusion (26) sont alignées à intervalles réguliers dans le sens de la largeur (4Y) du joint d'étanchéité latéral (4).

9. Couche-culotte jetable selon la revendication 8, où un pas d'agencement (PS) des zones de liaison par fusion (26) dans le sens de la largeur (4Y) du joint d'étanchéité latéral (4) est au moins deux fois supérieur à la longueur de la zone de liaison par fusion (26) dans le sens de la largeur (4Y).

10. Couche-culotte jetable selon l'une des revendications 1 à 10, où ladite couche-culotte jetable comprend une partie où la zone de raccordement et la zone de liaison par fusion se chevauchent l'une l'autre.

11. Couche-culotte jetable selon l'une des revendications 1 à 10, où une longueur de la zone de raccordement (40) dans le sens de la longueur (4X) du joint d'étanchéité latéral (4) est inférieure à la longueur de la zone de raccordement (40) dans le sens de la largeur (4Y) du joint d'étanchéité latéral (4).

12. Couche-culotte jetable selon l'une des revendications 1 à 11, où les joints d'étanchéité latéraux comprennent la zone de liaison par fusion dans au moins une zone sur le côté de l'ouverture de taille entre deux zones définies par partage en deux de l'ensemble d'un joint d'étanchéité latéral dans le sens de la longueur.

13. Couche-culotte jetable selon l'une quelconque des revendications 1 à 12, où un pas d'agencement (PS) des zones de liaison par fusion (26) dans le sens de la longueur (4X) du joint d'étanchéité latéral (4) est inférieur au pas d'agencement (P1) des zones de raccordement (4) dans le sens de la longueur (4X) du joint d'étanchéité latéral (4).

14. Couche-culotte jetable selon l'une des revendications 1 à 13, où, dans un état plat et non contracté de la couche-culotte jetable, un pas d'agencement (PS) des zones de liaison par fusion (26) dans le sens de la largeur (Y) de la couche-culotte jetable est plus court, sur la périphérie du joint d'étanchéité latéral (4), que dans une partie autre que la périphérie du joint d'étanchéité latéral (4).

15. Couche-culotte jetable selon la revendication 14, où, dans un état plat et non contracté de couche-culotte jetable, un pas d'agencement (PS) des zones de liaison par fusion (26) dans le sens de la largeur (Y) de la couche-culotte jetable est plus court, dans une zone comprise dans une plage de 50 mm depuis une partie d'extrémité extérieure de la couche-culotte jetable dans le sens de la largeur (Y) vers l'intérieur dans le sens de la largeur (Y) de la couche-culotte jetable, que dans une partie extérieure à la plage.
